# FASCICULE DE BREVET EUROPEEN

(11) **EP 0 988 079 B1**
(45) Date de publication et mention de la délivrance du brevet: **01.10.2003**
(21) Numéro de dépôt: 98929545.6
(22) Date de dépôt: 10.06.1998
(51) Int. Cl.: A61M 15/08

(54) **EMBOUT NASAL ET DISPOSITIF DE DISTRIBUTION COMPORTANT UN TEL EMBOUT**
NASAL-APPLIKATOR UND ABGABEVORRICHTUNG MIT DEMSELBEN
NASAL APPLICATOR AND DEVICE FOR DISPENSING DEVICE COMPRISING SAME

(30) Priorité: 18.06.1997 FR 9707585
(43) Date de publication de la demande: 29.03.2000
(73) Titulaire: VALOIS S.A.S., 27110 Le Neubourg (FR)
(72) Inventeur: OECHSEL, François, F-27400 Louviers (FR)
(74) Mandataire: Riege, Christian
(86) Numéro de dépôt international: FR9801196
(87) Numéro de publication internationale: WO98057690

(56) Documents cités:
- EP-A- 0 170 198
- FR-A- 1 257 220
- FR-A- 2 739 294

## Description

La présente invention concerne un embout nasal et un dispositif de distribution nasale d'un produit fluide ou pulvérulent comportant un tel embout. Plus particulièrement, l'invention concerne un embout de ce type adapté à définir la direction de distribution du produit dans la narine de l'utilisateur.

Les dispositifs de distribution nasale sont largement répandus et utilisés dans des applications diverses. Ces dispositifs comportent tous une tête incorporant l'orifice de distribution du produit, au moins une partie de ladite tête étant destinée à être insérée dans la narine de l'utilisateur lors de la distribution du produit. L'utilisateur actionne ensuite le dispositif pour expulser une dose de produit dans la narine.

Dans certaines applications, notamment dans le domaine de la pharmacie, les produits distribués par les dispositifs de distribution nasale peuvent être coûteux et/ou doivent être dosés de manière très précise. On recherche par conséquent à obtenir une efficacité maximale de la dose de produit distribué dans la narine à chaque actionnement.

Actuellement, la plupart des dispositifs de distribution nasale expulse la dose de produit dans une direction très sensiblement parallèle à la cloison nasale, vers le cornet supérieur de la narine.

Or, -il s'est avéré que pour un certain nombre de produits, notamment dans le domaine de la pharmacie, la partie la plus efficace pour l'assimilation du produit est constituée par le cornet inférieur de la narine.

Il existe sur le marché des distributeurs pourvus de poussoir (ou tête de distribution) incliné pour tenter de résoudre ce problème. Toutefois, ce type de poussoir incliné est compliqué et cher à fabriquer, notamment en ce qui concerne le moulage. De plus, l'efficacité de tels poussoirs n'est pas garantie. En outre, l'utilisation d'un poussoir incliné (ou coudé) implique la présence d'un canal d'expulsion relativement long et empêche l'utilisation de gicleurs internes. Or, les gicleurs internes fournissent de nombreux avantages. Notamment, ils ne risquent pas de se détacher du poussoir (contrairement aux gicleurs externes) et ils permettent de minimaliser efficacement le volume mort à l'intérieur du poussoir.

Le document FR-2 739 294 divulgue une telle tête inclinée. Ce document divulgue en outre l'utilisation d'un embout de sécurité destiné à limiter à une longueur prédéterminée la partie de l'embout pénétrant dans la cavité nasale, afin d'éviter un contact traumatisant entre l'embout et les muqueuses de la narine.

C'est un but de la présente invention que de fournir un embout et un dispositif de distribution nasale qui ne reproduisent pas les inconvénients précités.

Plus particulièrement, c'est un but de la présente invention que de fournir un embout nasal qui permet, lors de l'actionnement du dispositif de distribuer le produit dans la zone de la fosse nasale où l'assimilation du produit est la plus efficace.

C'est aussi un but de l'invention que de fournir un tel dispositif de distribution nasale qui ne comporte pas les inconvénients des poussoirs inclinés ou coudés.

C'est également un but de la présente invention que de fournir -un dispositif de distribution nasale du type précité qui soit simple et peu coûteux à fabriquer et à assembler. Ceci est notamment le cas dans certains cas d'applications particuliers où le dispositif de distribution nasale n'est destiné qu'à expulser un très petit nombre de doses de produit.

C'est également un but de la présente invention que de fournir un tel dispositif de distribution nasale qui soit d'utilisation simple et fiable pour n'importe quel utilisateur.

La présente invention a donc pour objet un embout nasal adapté à coopérer avec une tête d'un dispositif de distribution nasale d'un produit fluide ou pulvérulent, ladite tête étant destinée à être au moins partiellement insérée dans une narine lors de l'actionnement du dispositif, caractérisé en ce que ledit embout coopère avec l'extrémité de la tête, ledit embout comportant un appui s'étendant environ transversalement à la direction de distribution du produit, ledit appui étant adapté à s'appuyer sous la narine sur la lèvre supérieure de l'utilisateur pour définir l'angle d'insertion de ladite tête dans la narine.

Selon un mode de réalisation avantageux de l'invention, ledit embout comporte un anneau emmanchable autour de l'extrémité de la tête, ledit appui étant relié audit anneau.

Avantageusement, l'embout comporte un cylindre creux destiné à être emmanché autour de ladite extrémité de la tête, ledit appui se raccordant à un bout dudit cylindre.

De préférence, la longueur dudit cylindre détermine la profondeur de pénétration de la tête dans la narine, ledit appui formant butée sous la narine pour empêcher une pénétration plus profonde.

Selon une première variante de la présente invention, ledit appui dudit embout est réalisé sous la forme d'une plaque mince environ rectangulaire dont un bord a un profil adapté à s'appuyer sur la lèvre supérieure de l'utilisateur.

Selon une seconde variante de réalisation, ledit appui dudit embout est réalisé environ sous la forme d'un disque dont une partie de bord a un profil adapté à s'appuyer sur la lèvre supérieure et une partie de bord opposée a un profil adapté à s'appuyer sur le nez de l'utilisateur.

La présente invention a aussi pour objet un dispositif de distribution nasale d'un produit fluide ou pulvérulent, comportant un organe de distribution sur lequel est montée une tête pourvue d'un orifice de distribution, l'extrémité de ladite tête étant destinée à être au moins partiellement insérée dans une narine lors de l'actionnemen du dispositif, caractérisé en ce que le dispositif comprend un embout réalisé selon l'une quelconque des revendications 1 à 6.

Avantageusement, ladite tête comporte une extrémité cylindrique pourvue de l'orifice de distribution, ledit embout comportant un cylindre creux emmanché autour de ladite extrémité cylindrique de la tête jusqu'à un épaulement de butée disposé de telle sorte que le bout dudit cylindre est situé sensiblement au niveau dudit orifice de distribution.

Selon une variante de réalisation de la présente invention, ledit embout fait partie intégrante de ladite tête.

Selon une autre variante de réalisation de la présente invention, ledit embout est rapporté sur ladite tête.

De préférence, ledit embout et/ou ladite tête comporte(nt) des moyens pour déterminer l'orientation dudit embout par rapport à ladite tête.

D'autres caractéristiques et avantages de la présente invention apparaîtront au cours de la description détaillée suivante, de diverses variantes de réalisation, données à titre d'exemples non limitatifs, en référence aux dessins joints, sur lesquels :
- la figure 1 est une vue schématique en perspective montrant une tête et un embout selon un mode de réalisation particulier de la présente invention ; et
- les figures 2 à 5 sont des vues schématiques en perspective de diverses variantes de réalisation de l'embout selon la présente invention.

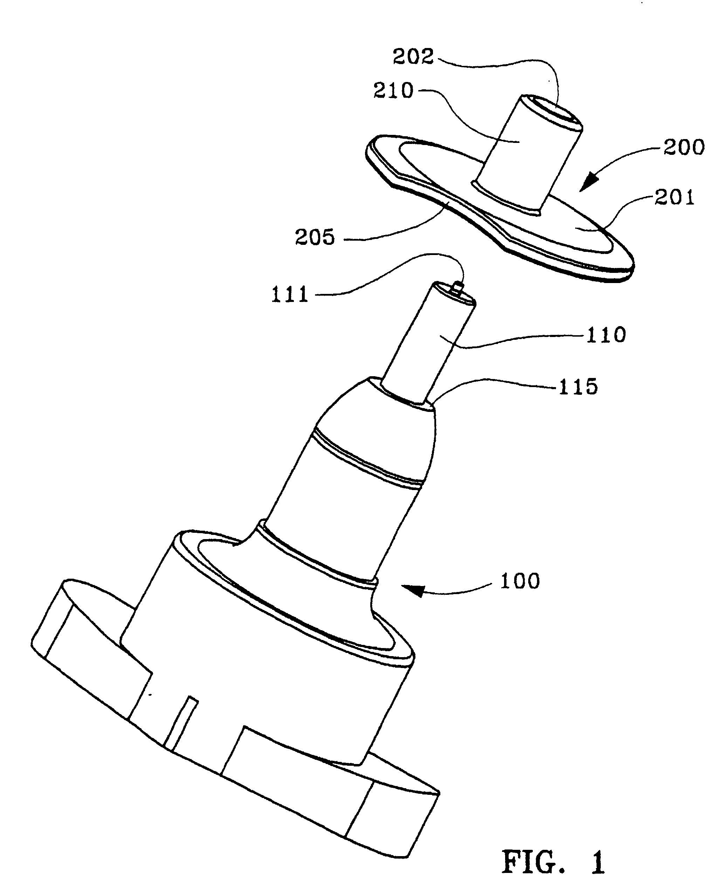

En référence aux figures 1 à 5, le dispositif de distribution nasale comporte une tête 100 (ou poussoir) montée sur un organe de distribution (non représenté), tel qu'une pompe ou une valve), ledit organe de distribution étant lui-même de manière classique monté sur un récipient contenant le produit (également non représenté). La présente invention s'applique en fait à tous les dispositifs de distribution nasale, et seules les parties du dispositif intervenant dans la présente invention sont représentées sur les dessins et décrits ci-après.

Selon l'invention, un embout 200 est prévu pour coopérer avec l'extrémité 110 de la tête 100 du dispositif de distribution nasale, au moins une partie de ladite tête étant destinée à être insérée dans la narine lors de l'actionnement du dispositif.

Les diverses variantes de réalisation représentées sur les figures montrent toutes un embout 200 séparé de la tête 100 et qui est prévu pour être rapporté sur ladite tête avant l'utilisation du dispositif. Il doit toutefois être clair que ces variantes correspondent à un mode de réalisation préféré de l'invention. En effet, l'invention concerne également un tel embout qui ferait partie intégrante de la tête, la tête et l'embout pouvant soit être moulés d'une pièce, soit être fixés l'un à l'autre lors du montage du dispositif de distribution.

De même, lorsque l'embout nasal est rapporté, il peut être adapté sur la tête de manière amovible ou de manière fixe. Un embout rapporté n'est par conséquent pas nécessairement un embout amovible.

Selon l'invention, ledit embout 200 comporte un appui 201 qui s'étend environ transversalement à la direction de distribution du produit lorsque l'embout est disposé sur la tête de distribution 100 du dispositif. Plus particulièrement, dans l'exemple représenté sur la figure 1, l'appui 201 s'étend perpendiculairement à l'axe principal longitudinal de la tête 100, axe principal qui passe par l'orifice de distribution 111.

Avantageusement, ledit embout 200 est destiné à être emmanché autour de l'extrémité 110 de la tête 100. Dans ce cas, ladite tête 100 comporte avantageusement un épaulement de butée 115 qui limite l'emmanchement dudit embout 200. En variante, notamment dans le cas d'une tête de forme conique, cet épaulement de butée 115 n'est pas nécessaire, l'arrêt de l'emmanchement de l'embout se faisant automatiquement par l'augmentation du diamètre extérieur de ladite tête 100.

Avantageusement, la tête 100 et/ou l'embout 200 comporte(nt) l'un ou l'autre (ou tous les deux) des moyens appropriés (non représentés) pour prédéterminer l'orientation de l'embout 200 par rapport à ladite tête 100 lors de l'emmanchement de l'un sur l'autre. Ces moyens d'orientation peuvent être réalisés sous la forme de méplats ou de rainures, ou par tout autre moyen approprié.

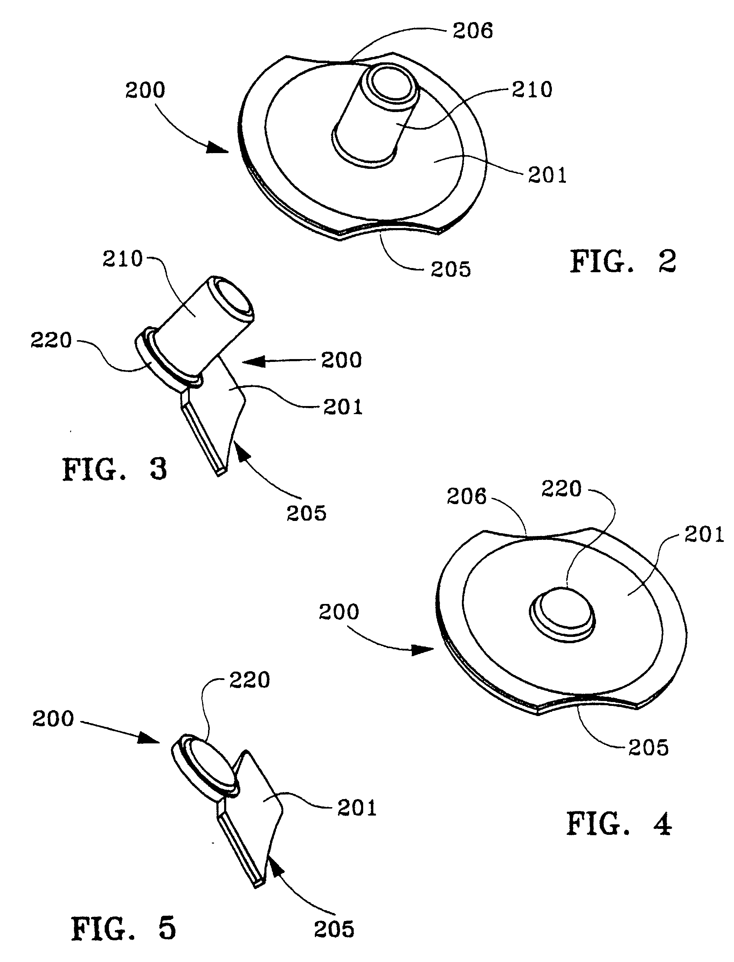

Les figures 2 à 5 représentent diverses variantes de réalisation de l'embout 200. Ainsi, en référence aux figures 4 et 5, l'embout comporte un anneau 220 emmanchable autour de l'extrémité 110 de la tête de distribution 100, ledit appui 201 étant relié audit anneau 220. Dans les exemples représentés sur les figures 2 et 3, ledit anneau 220 se prolonge par un cyclindre creux 210 destiné à être emmanché autour de ladite extrémité 110 de la tête de distribution 100, la longueur dudit cylindre creux déterminant avantageusement la profondeur de pénétration de la tête 100 dans la narine. L'appui 201 forme avantageusement une butée sous la narine pour empêcher une pénétration plus profonde.

Dans les variantes représentées sur les figures 3 et 5, l'appui 201 de l'embout 200 est réalisé sous la forme d'une plaque mince, avantageusement environ rectangulaire, qui vient s'appuyer sur la lèvre supérieure de l'utilisateur. Pour ce faire, l'appui 201 comprend de préférence un bord 205 avec un profil adapté à la forme de la lèvre supérieure. Dans les exemples représentés sur les figures 1, 2 et 4, ledit appui est réalisé sous la forme générale d'un disque 201. Dans ce cas, une partie de bord 205 dudit disque vient s'appuyer sur la lèvre supérieure et une partie de bord opposée 206 vient s'appuyer sur le nez de l'utilisateur. De manière similaire, ladite partie de bord 205 et/ou ladite partie de bord opposée 206 peuvent comporter un profil adapté d'une part à la forme de la lèvre supérieure et d'autre part à la forme du nez.

D'autres variantes de réalisation sont imaginables pour réaliser la fonction revendiquée par la présente invention.

Le fonctionnement du dispositif est donc le suivant.

Si l'embout 200 est réalisé de manière séparée de la tête de distribution 100, l'utilisateur emmanche préalablement ledit embout 200 sur l'extrémité 110 de la tête 100. Le dispositif de distribution nasale comporte ainsi une partie radialement saillante formée par ledit appui 201. L'utilisateur insère alors la tête 100, du moins son extrémité 110, dans sa narine. Pendant cette opération, l'appui 201 de l'embout 200 vient s'appuyer contre la lèvre supérieure, obligeant l'utilisateur à pivoter le dispositif de distribution vers une direction proche de l'horizontale pour pouvoir l'insérer dans la narine. Par conséquent, l'utilisateur est fortement incité d'insérer la tête de distribution dans une direction proche de l'horizontale. Selon la variante de réalisation de l'embout utilisée, l'appui 201 vient en appui soit uniquement sur la lèvre supérieure, soit à la fois sur la lèvre supérieure et sur l'aile du nez, pour définir d'une part la direction d'insertion de la tête dans la narine, et donc la direction de distribution du produit dans la narine, et d'autre part, la profondeur d'insertion de ladite tête 100 dans la narine. L'utilisateur actionne alors le dispositif et la dose de produit est distribuée dans la zone souhaitée, à savoir au niveau du cornet inférieur de la narine.

Un avantage particulier de l'invention est de pouvoir assurer une distribution efficace du produit tout en limitant les coûts de fabrication du dispositif. En effet, l'embout de l'invention est très facilement moulable et s'adapte sur une tête de distribution qui n'a pas besoin d'être inclinée. Les complications de fabrication engendrées par de telles têtes inclinées sont donc évitées.

Le dispositif de la présente invention comporte également les avantages supplémentaires suivants :
- le dispositif de distribution nasale de la présente invention s'adapte facilement à l'utilisation simultanée chez les enfants et les adultes. Pour ce faire, il suffit de prévoir un embout 200 avec un cylindre 210 plus ou moins long correspondant à la distance optimale d'insertion dans la narine selon que l'utilisateur est un enfant ou un adulte ;
- dans le cas où l'embout 200 vient s'emmancher autour de l'extrémité 110 de la tête 100, et que l'embout 200 comporte un cylindre creux 210, l'extrémité 210 de la tête peut être réalisée de manière cylindrique. Ceci permet une simplification du moulage de la partie haute du poussoir nasal, en particulier, au niveau de l'orifice de distribution 111. Dans l'exemple représenté sur la figure 1, la partie de la tête, dans laquelle se fait la jonction entre la paroi extérieure de la tête et sa structure intérieure (dans laquelle vient notamment se fixer le gicleur interne), peut être décalée en éloignement de l'orifice de distribution, à savoir au niveau de l'épaulemen de butée 115. Ainsi, des défauts de moulage au niveau de cette jonction n'influencent plus de manière négative la pulvérisation du produit qui se fait au niveau de l'orifice de distribution.

La présente invention permet donc de fournir un dispositif de distribution nasale simple et peu coûteux à fabriquer (notamment à mouler) et à assembler. Ce dispositf est en outre simple et fiable à utiliser par tout le monde, l'appui 201 de l'embout incitant, voire obligeant, l'utilisateur à insérer la tête de l'appareil dans la narine dans la direction souhaitée pour distribuer la dose de produit dans la zone souhaitée de la narine. Ceci est particulièrement avantageux dans le domaine de la pharmacie où le dosage du produit doit être très précis et son efficacité optimisée en raison du coût souvent élevé des produits à distribuer.

## Revendications

1. Embout nasal adapté à coopérer avec une tête (100) d'un dispositif de distribution nasale d'un produit fluide ou pulvérulent, ladite tête (100) étant destinée à être au moins partiellement insérée dans une narine lors de l'actionnement du dispositif, **caractérisé en ce que** ledit embout (200) coopère avec l'extrémité (110) de la tête (100), ledit embout (200) comportant un appui (201) s'étendant environ transversalement à la direction de distribution du produit, ledit appui (201) étant adapté à s'appuyer sous la narine sur la lèvre supérieure de l'utilisateur pour définir l'angle d'insertion de ladite tête (100) dans la narine.

2. Embout nasal selon la revendication 1, dans lequel ledit embout (200) comporte un anneau (220) emmanchable autour de l'extrémité (110) de la tête (100), ledit appui (201) étant relié audit anneau (220).

3. Embout nasal selon la revendication 1 ou 2, dans lequel ledit embout (200) comporte un cylindre creux (210) destiné à être emmanché autour de ladite extrémité (110) de la tête (100), ledit appui (201) se raccordant à un bout dudit cylindre (210).

4. Embout nasal selon la revendication 3, dans lequel la longueur dudit cylindre (210) détermine la profondeur de pénétration de la tête (100) dans la narine, ledit appui (201) formant butée sous la narine pour empêcher une pénétration plus profonde.

5. Embout nasal selon l'une quelconque des revendications précédentes, dans lequel ledit appui (201) dudit embout (200) est réalisé sous la forme d'une plaque mince environ rectangulaire dont un bord (205) a un profil adapté à s'appuyer sur la lèvre supérieure de l'utilisateur.

6. Embout nasal selon l'une quelconque des revendications 1 à 4, dans lequel ledit appui (201) dudit embout (200) est réalisé environ sous la forme d'un disque dont une partie de bord (205) a un profil adapté à s'appuyer sur la lèvre supérieure et une partie de bord opposée (206) a un profil adapté à s'appuyer sur le nez de l'utilisateur.

7. Dispositif de distribution nasale d'un produit fluide ou pulvérulent, comportant un organe de distribution sur lequel est montée une tête (100) pourvue d'un orifice de distribution (111), l'extrémité (110) de ladite tête (100) étant destinée à être au moins partiellement insérée dans une narine lors de l'actionnement du dispositif, **caractérisé en ce que** le dispositif comprend un embout (200) réalisé selon l'une quelconque des revendications 1 à 6.

8. Dispositif selon la revendication 7, dans lequel ladite tête (100) comporte une extrémité cylindrique (110) pourvue de l'orifice de distribution (111), ledit embout (200) comportant un cylindre (210) creux emmanché autour de ladite extrémité cylindrique (110) de la tête jusqu'à un épaulement de butée (115) disposé de telle sorte que le bout dudit cylindre (210) est situé sensiblement au niveau dudit orifice de distribution (111).

9. Dispositif selon la revendication 7 ou 8, dans lequel ledit embout (200) fait partie intégrante de ou est fixée à ladite tête (100).

10. Dispositif selon la revendication 7 ou 8, dans lequel ledit embout (200) est rapporté sur ladite tête (100).

11. Dispositif selon l'une quelconque des revendications 7 à 10, dans lequel ledit embout (200) et/ou ladite tête (100) comporte(nt) des moyens pour déterminer l'orientation dudit embout (200) par rapport à ladite tête (100).

## Patentansprüche

1. Nasal-Applikator, der geeignet ist, mit einem Kopf (100) einer Nasal-Abgabevorrichtung für ein fluid- oder pulverförmiges Produkt zusammenzuwirken, wobei der Kopf (100) dazu bestimmt ist, bei der Betätigung der Vorrichtung zumindest teilweise in ein Nasenloch eingeführt zu werden, **dadurch gekennzeichnet, dass** der Applikator (200) mit dem Ende (110) des Kopfes (100) zusammenwirkt, wobei der Applikator (200) ein Abstützteil (201) umfaßt, das sich ungefähr quer zur Abgaberichtung des Produktes erstreckt, wobei das Abstützteil (201) geeignet ist, sich unter dem Nasenloch auf der Oberlippe des Verwenders abzustutzen, um den Einführwinkel des Kopfes (100) in das Nasenloch zu definieren.

2. Nasal-Applikator nach Anspruch 1, bei dem der Applikator (200) einen um das Ende (110) des Kopfes (100) aufsetzbaren Ring (220) umfaßt, wobei das Abstützteil (201) mit dem Ring (220) verbunden ist.

3. Nasal-Applikator nach Anspruch 1 oder 2, bei dem der Applikator (200) einen hohlen Zylinder (210) umfaßt, der dazu dient, um das Ende (110) des Kopfes (100) herum aufgesetzt zu werden, wobei das Abstützteil (201) mit einem Ende des Zylinders (210) verbunden ist.

4. Nasal-Applikator nach Anspruch 3, bei dem die Länge des Zylinders (210) die Eindringtiefe des Kopfes (100) in das Nasenloch bestimmt, wobei das Abstützteil (201) einen Anschlag unter dem Nasenloch bildet, um ein noch tieferes Eindringen zu verhindern.

5. Nasal-Applikator nach einem der vorhergehenden Ansprüche, bei dem das Abstützteil (201) des Applikators (200) in Form einer kleinen, ungefähr rechtwinkeligen Platte ausgebildet ist, deren Rand (205) ein Profil besitzt, das geeignet ist, sich an der Oberlippe des Verwenders abzustützen.

6. Nasal-Applikator nach einem der Ansprüche 1 bis 4, bei dem das Abstützteil (201) des Applikators (200) ungefähr in der Form einer Scheibe ausgebildet ist, von der ein Randteil (205) ein Profil aufweist, das geeignet ist, sich auf der Oberlippe des Verwenders abzustützen und einen gegenüberliegenden Randteil (206) mit einem Profil, das geeignet ist, sich an der Nase des Verwenders abzustützen.

7. Nasal-Abgabevorrichtung für ein fluid- oder pulverförmiges Produkt, die ein Abgabeorgan umfaßt, auf dem ein Kopf (100) montiert ist, der mit einer Abgabeöffnung (111) versehen ist, wobei das Ende (110) des Kopfes (100) dazu bestimmt, ist, bei der Betätigung der Vorrichtung zumindest teilweise in ein Nasenloch eingeführt zu werden, **dadurch gekennzeichnet, dass** die Vorrichtung einen Applikator (200) umfaßt, der gemäß einem der Ansprüche 1 bis 6 ausgebildet ist.

8. Vorrichtung nach Anspruch 7, bei der der Kopf (100) ein zylindrisches Ende (110) umfaßt, das mit einer Abgabeöffnung (111) versehen ist, wobei der Applikator (200) einen hohlen Zylinder (210) aufweist, der um das zylindrische Ende (110) des Kopfes bis zu einer Anschlagsschulter (115) aufgesetzt ist, die derart angeordnet ist, dass sich das Ende des Zylinders (210) im wesentlichen in Höhe der Abgabeöffnung (111) befindet.

9. Vorrichtung nach Anspruch 7 oder 8, bei der der Applikator (200) einen integralen Teil des Kopfes (100) bildet oder an diesem befestigt ist.

10. Vorrichtung nach Anspruch 7 oder 8, bei der der Applikator (200) auf den Kopf (100) aufgesetzt ist.

11. Vorrichtung nach einem der Ansprüche 7 bis 10, bei der der Applikator (200) und/oder der Kopf (100) Mittel umfaßt bzw. umfassen, die dazu dienen, die Ausrichtung des Applikators (200) bezüglich des Kopfes (100) festzulegen.

## Claims

1. A nasal applicator suitable for co-operating with a head (100) of a nasal dispenser device for dispensing a fluid or powder substance into the nose, said head (100) being designed to be inserted at least in part into a nostril when the device is actuated, said nasal applicator being **characterized in that** said applicator (200) co-operates with the end (110) of the head (100), said applicator (200) being provided with a bearing piece (201) that extends substantially transversely to the direction in which the substance is dispensed, said bearing piece (201) being suitable for coming to bear under the nostril against the top lip of the user so as to define an insertion angle at which said head (100) is inserted into the nostril.

2. A nasal applicator according to claim 1, in which said applicator (200) includes a ring (220) that can fitted around the end (110) of the head (100), said bearing piece (201) being connected to said ring (220).

3. A nasal applicator according to claim 1 or 2, in which said applicator (200) includes a hollow cylinder (210) designed to be fitted around said end (110) of the head (100), said bearing piece (201) being connected to one end of said cylinder (210).

4. A nasal applicator according to claim 3, in which the length of said cylinder (210) determines the depth to which the head (100) penetrates into the nostril, said bearing piece (201) forming an abutment under the nostril to prevent said head from penetrating any deeper.

5. A nasal applicator according to any preceding claim, in which said bearing piece (201) on said applicator (200) is implemented in the form of a substantially rectangular thin plate having and edge (205) which has a profile suitable for bearing against the top lip of the user.

6. A nasal applicator according to any one of claims 1 to 4, in which said bearing piece (201) of said applicator (200) is implemented substantially in the form of a disk having an edge portion (205) that has a profile suitable for bearing against the top lip, and an opposite edge portion (206) having a profile suitable for bearing against the nose of the user.

7. A nasal dispenser device for dispensing a fluid or powder substance into the nose, said device including a dispenser member on which a head (100) provided with a dispenser orifice (111) is mounted, the end (110) of said head (100) being designed to be inserted at least in part in a nostril when the device is actuated, said device being **characterized in that** it includes an applicator (200) according to any one of claims 1 to 6.

8. A device according to claim 7, in which said head (100) includes a cylindrical end (110) provided with the dispenser orifice (111), said applicator (200) including a hollow cylinder (110) fitted around said cylindrical end (110) of the head by being engaged to an abutment shoulder (115) disposed such that the end of said cylinder (210) is situated substantially at said dispenser orifice (111).

9. A device according to claim 7 or 8, in which said applicator (200) is an integral part of or is fixed to said head (100).

10. A device according to claim 7 or 8, in which said applicator (200) is separate and is mounted on said head (100).

11. A device according to any one of claims 7 to 10, in which said applicator (200) and/or said head (100) is/are provided with means for determining the angular position of said applicator (200) relative to said head (100).
